# NEW EUROPEAN PATENT SPECIFICATION

(11) **EP 0 266 119 B2**
(45) Date of publication and mention of the opposition decision: **04.05.2005**
(45) Mention of the grant of the patent: 20.07.1994
(21) Application number: 87309286.0
(22) Date of filing: 21.10.1987
(51) Int. Cl.: A61K 9/50, A61K 9/56, A61K 9/62

(54) **Method and formulation for orally administering bioactive agents to and through the Peyer's patch**
Methode und Zusammensetzung zur oralen Verabreichung von bioaktiven Mitteln zur Peyer's Flecken
Méthode et formulation pour l'administration orale d'agents biologiquement actifs dirigés par les taches de Peyer

(30) Priority: 24.10.1986 US 923159
(43) Date of publication of application: 04.05.1988
(73) Proprietor: SOUTHERN RESEARCH INSTITUTE, Birmingham Alabama 35255-5305 (US); THE UAB RESEARCH FOUNDATION, Birmingham Alabama 35294 (US)
(72) Inventor: Tice,Thomas R., Birmingham,Alabama 35216 (US); Staas,Jay K., Pinson,Alabama 35126 (US); Gilley, Richard M., Birmingham, Alabama 35243 (US); Eldridge,John H., Birmingham,Alabama 35216 (US)
(74) Representative: Leissler-Gerstl, Gabriele

(56) References cited:
- EP-A- 0 058 481
- EP-A- 0 126 537
- EP-A- 0 130 162
- EP-A- 0 142 192
- US-A- 3 823 228
- US-A- 3 823 228
- US-A- 4 107 288
- US-EP- RE3 823 228
- JOURNAL OF BIOENGINEERING, vol. 1, January 1976, pp. 25-31; Thomas M.S. Chang: "Biodegrable semipermeable microcapsules containing enzymes, hormones, vaccines, and other biologicals"
- JOURNAL OF PHARMACEUTICAL SCIENCES, vol. 73, no. 11, November 1984, Washington D.C. US; P. Artursson et al.: "Characterization of polyacryl starch microparticles as carriers for proteins and drugs"
- Oral Induction of the Secretory Antibody Response by Soluble and Particulate Antigens ; Int. Archs. Allergy Appl. Immun. 75, 126-130.
- Controlled Release of a luteinizing Hormone-Releasing Hormone Analogue from Poly(d,l-lactide-co-glycolide)Microspheres; Sanders, L.M. et al. J. Pharm. Sci. 73, 1294, 1984.
- Cox and Taubman, Int. Archs Allergy Immunol, Vol. 75, pp. 126-131 (1984)
- Sanders et al, J. Pharm. Sci., vol. 73, n°9, pp. 1294-1297 (1984)
- Lefevre et al., J. Tox and Envir. Health, Vol. 6, pp. 691-704 (1980)
- Kreuter et al, Inf. and Imm., vol. 13, n°1, pp. 204-210 (1976)
- Artusson et al, J. Pharm. & Exp. Thera., vol. 234, n°1, pp. 255-260 (1985)
- Preis et al, J. Immunol. Meth., vol. 28, pp. 293-197 (1979)
- Declaration of Derek Thomas O'Hagan
- Chang J. Bioeng. Vol. 1, pp. 25-32 (1976)
- Cox and Muench, Int. Archs Allergy Appl. Immunol., vol. 74, pp. 249-255 (1984)
- Kreuter et al, J. Pharm. Sci., vol. 70, n°4, pp. 367-371 (1981)
- Deasy, "Microencapsulation and Related Drug Processes", Drugs andthe Pharmaceutical Sciences, pp. 8-13 and 219-227 (1984)
- Saffran et al, Science, vol. 233, pp. 1081-1084 (1986)
- Lazzell et al, J. Biol. Stand. Vol. 12, pp. 315-321 (1984)
- Black et al,Dev. Stand., Vol. 53, pp. 9-14 (1984)
- Shigeta et al, Endocrinol., Vol. 91, pp. 320-322 (1972)
- O'Hagan et al, Critical Reviews in Therapeutic Drug Carrier Systems, Vol. 4, pp. 197-220 (1987)
- O'Hagan et al, Pharmaceutical Formulations as Immunological Adjuvants, Thesis, University of Nottingham (December 1987)
- O'Hagan et al, Poly(butyl-2-cyanoacrylate)Particles as Adjuvants for Otal Immunization, Vaccine, Vol. 7, pp. 213-216 (June 1989)
- O'Hagan et al, Microparticles as Potentially Orally Active Immunological Adjuvants, Vaccine, Vol. 7, pp. 421-424 (October 1989)
- Challacombe et al, Induction of Secretory IgA Antibodies by Oral Immunisation with Biodegradable Microparticles, 7th International Congress of Musocal Immunology, Abstract Book, Prague, p. 41 (August 16-20 1992)
- O'Hagan et al, Biodegradable Microparticles as Oral Vaccines, 7th Innternational Congress of mucosal Immunology, Abstract Book, Prague, p. 175 (August 16-20 1992)
- Eldridge et al, Biodegradable Microspheres : Vaccine Delivery System for Oral Immunization, Current Topics in Microbiology and Immunology, Vol. 146, pp. 59-66 (1989)
- Ebel (1990) Pharm. Res. 7, 848-851
- Declaration of Dr. Thomas R. Tice and attachments re Pennwalt Award
- Declaration of Dr. Herbert M. Blatter re licensing
- Declaration of Mr. Martyn D. Greenacre re licensing
- Declaration of Dr. Thomas R. Tice and attachments re Peyer's patch microsphere vaccine publications chronology
- Declaration of Dr. Thomas R. Tice re Patent Proprietor and O'Hagan disclosure chronoloy
- Declaration of Paul J. Wilson
- Declaration of D.T. O'Hagan, 21.3.1997
- Declaration of Stanley Stewart Davis
- Declaration of Ruth Duncan
- Declaration of Martin Taubman

## Description

This invention relates to a method for orally administering an antigen encapsulated in one or more biodegradable, biocompatible polymer or copolymer excipients which results in the agent reaching and being taken up by the folliculi lymphatic aggregati, otherwise known as the "Peyer's patch", of the animal without loss of effectiveness due to the antigen having passed through the gastrointestinal tract.

The invention also relates to formulation for use in the method of the invention.

The use of microencapsulation to protect sensitive bioactive agents from degradation has become well-known. Typically, a bioactive agent is encapsulated within a protective wall material, usually polymeric in nature. The agent to be encapsulated can be coated with a single wall of polymeric material, or can be homogeneously dispersed within a polymeric matrix. The amount of agent inside the microcapsule can be varied as desired, ranging from either a small amount to as high as 95% of the microcapsule composition. The diameter of the microcapsule can also be varied as desired, ranging from less than one micrometer to as large as three millimetres or more.

Peyer's patches are conglomerations of lymphoid nodules located in the ileum or lower part of the intestine, and are an important part of the body's defense against bacterial infection. Antigens are substances that promote antibody formation, and include such things as foreign protein or tissues. All antibodies belong to a class of proteins called immunoglobulins (Ig). When an antibody and antigen combine, they form an inactive complex, thus neutralizing the antigen.

Peyer's patches possess IgA precursor B cells which can populate the lamina propria regions of the gastrointestinal and upper respiratory tracts and differentiate into mature IgA synthesizing plasma cells. It is these plasma cells which actually secrete the antibody molecules. Studies by Heremans and Bazin measuring the development of IgA responses in mice orally immunized with antigen showed that a sequential appearance of antigen-specific IgA plasma cells occurred, first in mesenteric lymph noces, later in the spleen, and finally in the lamina propria of the gastrointestinal tract (Bazin, H., Levi, G., and Doria, G. Predominant contribution of IgA antibody-forming cells to an immune response detected in extraintestinal lymphoid tissues of germfree mice exposed to antigen via the oral route. J. Immunol. 105: 1049; 1970 and Crabbe, P.A., Nash, D.R., Bazin, H. Eyssen, H., and Heremans. J.F. Antibodies of the IgA type in intestinal plasma cells of germfree mice after oral or parenteral immunization with ferritin. J. Exp. Med. 130: 723: 1969). It is apparent, therefore, that Peyer's patches are enriched sources of precursor IgA cells, which, subsequent to antigen sensitization, follow a circular migrational pathway and account for the expression of IgA at distant mucosal surfaces. This circular pattern provides a common mucosal immune system by continually transporting sensitized B cells to mucosal sites for responses to gut-encountered environmental antigens and potential pathogens.

Of particular importance to the present invention is the ability of oral immunization to induce protective antibodies. It is known that the ingestion of antigens by animals results in the appearance of antigen-specific sIgA anti-bodies in bronchial or nasal washings. For example, studies with human volunteers show that oral administration of influenza vaccine is effective at inducing secretory anti-influenza antibodies in nasal secretions.

It is apparent that any method or formulation involving oral administration of an ingredient be of such design that will protect the antigen from degradation during its passage through the gastrointestinal tract. If not, the ingredient will reach the Peyer's patch, if at all, in inadequate quantity or ineffective condition. In unprotected form large quantities of the antigen must be ingested for an effective amount of the antigen to reach the Payer's patch. The result is that a large percentage of the administered antigen unused. Also, frequent oral administrations are necessary to achieve a prolonged delivery of antigen to the Peyer's patch. Such frequent administration of high doses of antigen is both wasteful and inconvenient.

Therefore, there exists a need for a method of oral immunization which will effectivelystimulate the mucosal immune system and overcome the problem of degradation of the bioactive ingredient during its passage through the gastrointestinal tract to the Peyer's patch. There exists a more particular need for a method of delivering an antigen to the Peyer's patch which does not result in degradation.

According to another aspect of the invention there is provided an oral composition for immunisation to be administered to animals, including humans, and capable of delivering an antigen to the Peyer's patch of said animal, comprising an effective amount of the antigen encapsulated in a biodegradable, biocompatible excipient so as to form microcapsules having a size less than or equal to 10µm being capable of being taken up selectively by the Peyer's patch and capable of passing through the gastrointestinal tract without degradation.

Preferably, the microcapsules are 1 to 10µm in size.

The antigen is microencapsulted in one or more biodegradable, biocompatible polymers or copolymers which is are capable of passing through the gastrointestinal tract without degredation or minimal degredation so that the antigen reaches the Peyer's patch unaltered and in effective amounts. The term biocompatible is defined as a polymeric material which is not toxic to the body, is not carcinogenic, and should not induce inflammation of body tissues. The material should be biodegradable in the sense that it should degrade by bodily processes to products readily disposable by the body and should not accumulate in the body. The excipient may be poly(DL lactide-co-glycolide). The copolymer excipient may have mole ratios of lactide to glycolide of 50:50 to 90:10 respectively. The microcapsule is also of a size capable of being selectively taken up by the selective Peyer's patch. Therefore the problems of the agent reaching the Peyer's patch and being taken up are solved.

It is an object of this invention to provide a composition which is as defined in claim 16 which can be orally administered to an animal which results in the agent reaching and being taken up by the Peyer's patch, and thereby stimulating the mucosal immune system, without losing its effectiveness as a result of passing through the animal's gastrointestinal tract.

It is a further object of this invention to provide a formulation comprising a core antigen and an encapsulating polymer or copolymer excipient which is biodegradable and biocompatible and which can be utilized in the oral administration methods described above.

An illustration of the method of performing one embodiment of the invention, that is, the prolonged delivery of the antigen trinitrophenyl keyhole limpet hemocyanin encapsulated in 50:50 poly (DL-lactide-co-glycolide) to mice follows.

It should be noted, however, that other polymers besides poly(DL-lactide-co-glycolide) may be used. Examples of such polymers include, but are not limited to, poly(glycolic acid), copolymers of mixed DL-lactide and glycolide, copolyoxalates, polycaprolactone, poly ( lactide-co-caprolactone), poly(esteramides), polyorthoesters and poly(β-hydroxybutyric acid).

Also, other antigen may be used. Examples of such, but not limited to include antigens to vaccinate against viral, bacterial, protozoan, fungal diseases such as influenza, respiratory syncytial, parainfluenza viruses. Hemophilus influenzae, Bordetella pertussis, Neisseria gonorrhoeae, Streptococcus pneumoniae and Plasmodium falciparum or other diseases caused by pathogenic microorganisms or antigens to vaccinate against diseases caused by microorganisms such as helminthic pathogens. The antigen may be a toxoid. The toxoid may be a toxoid of staphylococcal enterotoxin.

### I. MICROENCAPSULATIONS

### A. Preparation of Dye-Loaded Microcapsules for Peyer's-Patches Penetration Studies

Coumarin, a water-insoluble dye was microencapsulated with polystyrene, a nonbiodegradable polymer, to afford colourful microcapsules that could be used to follow the penetration of microcapsules into the Peyer's patches. The procedure used to prepare these microcapsules follows: First, a polymer solution is prepared by dissolving 4.95 g of polystyrene (Type 685D, Dow Chemical Company, Midland, MI) in 29.5 g of methylene chloride (Reagent Grade, Eastman Kodak, Rochester, NY). Next, about 0.05 g of coumarin (Polysciences. Inc., Warrington, PA) is added to the polymer solution and allowed to dissolve by stirring the mixture with a magnetic stir bar.

In a separate container, 10 wt-% aqueous poly(vinyl alcohol) (PVA) solution, the processing medium, is prepared by dissolving 40 g of PVA (Vinol 205C. Air Products and Chemicals, Allentown, PA) in 360 g of deionized water. After preparing the PVA solution, the solution is saturated with methylene chloride by adding 6 g of methylene chloride. Next, the PVA solution is added to 1-L resin kettle (Ace Glass, Inc., Vineland, NJ) fitted with a truebore stir shaft and a 2.5-in. Teflon (trademark) turbine impeller and stirred at about 380 rpm by a Fisher stedi speed motor.

The polystyrene/coumarin mixture is then added to the resin kettle containing the PVA processing media. This is accomplished by pouring the polystyrene/coumarin mixture through a long-stem 7-mm bore funnel which directs the mixture into the resin kettle. A stable oil-in-water emulsion results and is subsequently stirred for about 30 min at ambient pressure to afford oil microdroplets of the appropriate size. Then the resin kettle is closed; and the pressure in the resin kettle is gradually reduced to 520 mm Hg by means of a water aspirator connected to a manometer and bleed valve. The resin kettle contents are stirred at reduced pressure for about 24 h to allow all of the methylene chloride to evaporate. After all of the methylene chloride has evaporated, the hardened microcapsules are collected by centrifugation and dried for 72 h in a vacuum chamber maintained at room temperature.

### B Preparation of Antigen-Loaded Microcapsules

TNP-KLH, a water-soluble antigen, was encapsulated in poly(DL-lactide-co-glycolide), a biocompatible, biodegradable polyester. The procedure used to prepare the microcapsules follows:

First, a polymer solution was prepared by dissolving 0.5g of 50:50 poly(DL-lactide-co-glycolide) in 4.0 g of methylene chloride. Next, 300 microliter of an aqueous solution of TNP-KLH (46 mg TNP-KLH/mL; after dialysis) was added to and homogeneously dispersed in the poly(DL-lactide-co-glycolide) solution by vortexing the mixture with a Vortex-Genie 2 (Scientific Industries, Inc., Bohemia, NY).

In a separate container, a 8 wt % aqueous PVA solution was prepared by dissolving 4.8 g of PVA in 55.2 g of deionized water. After dissolution of the PVA, the PVA solution was added to a 100-mL resin kettle (Kontes Glass, Inc., Vineland, NJ) fitted with a truebore stirrer and a 1.5-in. Teflon turbine impeller. The polymer solution was then added to the PVA processing medium by pouring through a long-stem 7-mm bore funnel. During this addition, the PVA solution was being stirred at about 650 rpm. After the resulting oil-in-water emulsion was stirred in the resin kettle for about 10 min, the contents of the resin kettle contents were transferred to 3.5 L of deionized water contained in a 4-L beaker and being stirred at about 800 rpm with a 2-in. stainless steel impeller. The resultant microcapsules were stirred in the deionized water for about 30 min, collected by centrifugation, washed twice with deionized water to remove any residual PVA, and were then collected by freeze drying. The microcapsule products consisted of spherical particles about 1 to 10 micrometer in diameter.

The TNP-KLH content of the antigen-loaded microcapsules, that is, the core loading of the microcapsules, was determined by weighing out 10 mg of antigen-loaded microcapsules in a 12-mL centrifuge tube. Add 3.0 mL of methylene chloride to the tube and vortex to dissolve the poly(DL-lactide-co-glycolide). Next, add 3.0 mL of deionized water to the tube and vortex vigorously for 1 min. Centrifuge the contents of the centrifuge tube to separate the organic and aqueous layers. Transfer the aqueous layer to a 10-mL volumetric flask. Repeat the extraction combining the aqueous layers in the volumetric flask. Fill the flask to the mark with deionized water. The amount of TNP-KLH in the flask and subsequently, the amount of TNP-KLH in the microcapsules is then quantified using a protein assay. The microcapsules confined 0.2 % TNP-KLH by weight.

### II BIOLOGICAL STUDIES

### A. Mice

BALB.c mice. 8 to 12 weeks of age, were used in these studies.

### B. Trinitrophenyl - Keyhole Limpet Hemocyanin

Hemocyanin from the keyhole limpet (KLH) Megathura crenulate was purchased from Calbiochem (San Diego, CA). It was conjugated with the trinitrophenyl hapten (TNP-KLH) using 2,4,6-trinitrobenzene sulfonic acid according to the procdure of Rittenburg and Amkraut (Rittenburg, M.B. and Amkraut, A.A. Immunogenicity of trinitrophenyl-hemocyanin: Production of primary and secondary anti-hapten precipitins. J. Immunol. 97: 421: 1966). The substitution ratio was spectrophotomatrically determined to be TNP₈₆₁-KLH using a molar extinction coefficient of 15.400 at a wavelength of 350 nm and applying a 30% correction for the contribution of KLH at this wavelength (Rittenburg, M.B. and Amkraut, A.A. Immunogenicity of trinbitrophenyl-hemocyanin: Production of primary and secondary anti-hapten precipitins, J. Immunol. 97:421: 1966).

### C. Immunization

Microencapsulated and nonencapsulated TNP-KLH was suspended at an antigen concentration of 10 µg/mL in a solution of 8 parts filter sterilized tap water and 2 parts sodium bicarbonate (7.5% solution). The recipient mice were fasted overnight prior to the administration of 0.5 mL of suspension via gastric intubation carried out with an intubation needle (Babb, J.L., Kiyono, H., Michalek, S.M. and McGhee, J.R. LPS regulation of the immunce response: Suppression of immune responses to orally-administered T-dependent antigen. J. Immunol. 127: 1052: 1981).

### D. Collection of biological fluids

### 1. Serum

Blood was collected in calibrated capillary pipettes following puncture of the retro-orbital plexus. Following clot formation, the serum was collected, centrifuged to remove red cells and platelets. heat-inactivated, and stored at -70°C until assayed.

### 2. Intestinal secretions

Mice were administered four doses (0.5 mL) of lavage solution (25 mM NaC1, 40 mM Na₂SO₄, 10 mM KC1, 20 mM NaHCO₃, and 48.5 mM polyethylene glycol, osmolarity of 530 mosM) at 15-min intervals (Elson, C.O.. Ealding, W. and Lefkowitz. J. A lavage technique allowing repeated measurement of IgA antibody in mouse intestinal secretions. J. Immunol, Meth. 67: 101; 1984). Fifteen minutes after the last dose of lavage solution, the mice were anesthetized and after an additional 15 min they were administered 0.1 mg pilocarpine by ip injection. Over the next 10 to 20 min a discharge of intestinal contents was stimulated. This was collected into a petri dish containing 3 mL of a solution of 0.1 mg·mL soybean trypsin inhibitor (Sigma, St. Louis, MO) in 50 mM EDTA, vortexed vigorously and centrifuged to remove suspended matter. The supernatant was transferred to a round-bottom, polycarbonate centrifuge tube and 30 µL of phenylmethylsulfonyl fluoride (PMSF. Sigma) was added prior to clarification by high-speed centrifugation (27.000 x g. 20 min, 4°C). After clarification, 20 µL each of PMSF and 1% sodium azide were added and the solution made 10% in FCS to provide an alternate substrate for any remaining proteases.

### 3. Saliva

Concurrent with the intestinal discharge, a large volume of saliva is secreted and 0.25 mL was collected into a pasteur pipette by capillary action. Twenty microliters each of soybean trypsin inhibitor, PMSF, sodium aside and FCS was added prior to clarification.

### E. Immunochemical reagents

Solid-phase absorbed and affinity-purified polyclonal goat IgG antibodies specific for murine IgG and IgA were obtained commercially (Southern Biotechnology Associates. Birmingham. AL). Their specificity in radio-immunoassays were tested through their ability to bind appropriate monoclonal antibodies and myeloma proteins.

### F. Solid-phase radioimmunoassays

Purified antibodies were labelled with carrier-free Na¹²⁵I (Amersham) using the chloramine T method (Hunter, W.M. Radioimmunoassay. In: Handbook of Experimental Immunology, M.Weir (editor). Backwell Scientific Publishing, Oxford. p. 14.1; 1978). Immulon Removawell assay strips (Dynatech) were coated with TNP conjugated bovine serum albumin (BSA) at 1 µg/mL in BBS overnight at 4°C. Control strips were left uncoated but all strips were blocked for 2 h at room temperature with 1% BSA in BBS, which was used as the diluent for all samples and ¹²⁵I-labelled reagents. Samples of biologic fluids were diluted to contain 1 to 1.000 ng/mL of antigen-specific antibody of the isotype under study, added to washed triplicate replicate wells, and incubated 6 h at room temperature. After washing, 100,000 cpm of ¹²⁵I-labelled isotype-specific anti-immunoglobulin was added to each well and incubated overnight at 4 degrees Centigrade. Following the removal of unbound ¹²⁵I-antibodies by washing, the wells were counted in a Gamma 5500 spectrometer (Beckman Instruments, Inc., San Ramon, CA). Calibrations were made using serial twofold dilutions of a standard serum (Miles Scientific, Naperville, IL) containing known amounts of immunoglobulins, on wells coated with 1 µg/well isotype-specific antibodies. Calibration curves and interpolation of unknowns was obtained by computer, using "Logit-log" or "Four Parameter Logistic" BASIC programs (RIA 001 and RIA 004) available from the Biomedical Computing Technology Center (Vanderbilt Medical Center, Nashville. TN).

### G. RESULTS

### 1. Penetration of dye-loaded microcapsules into the Peyer's patches

The uptake of microcapsules into the gut-associated lymphoreticular tissues and the size restriction of this penetration was investigated by orally administering to mice polystyrene microcapsules, loaded with the fluorescent dye coumarin. Unanesthetized, fasted BALB/c mice were administered 0.5 mL of a 100 mg mL suspension of various sized fluorescent microcapsules (less than 5 µm or 8 to 50 µm in diameter) in tap water into the stomach using a feeding needle. At various times after administration (0.5, 1 and 2 h), the mice were sacrificed and the small intestine excised. One-cm sections of gut containing a discrete Peyer's patch were isolated, flushed of lumenal contents, everted, and snap frozen. Frozen sections were prepared and examined under a fluorescence microscope to observe the number, location and size of the microcapsules which were taken up into the Peyer's patch from the gut lumen.

Although some trapping of the microcapsules between the villi had prevented their removal during flushing, no penetration into the tissues was observed at any point except the Peyer's patch. At 0.5 h after oral administration, microcapsules were observed in the Peyer's patch of the proximal;, but not the distal, portion of the small intestine. With increasing time the microcapsules were transported by peristaltic movement such that by 2 h they were throughout the gastrointestinal tract and could be found in the Peyer's patch of the ileum. The endocytosed microcapsules were predominantly located peripherally, away from the apex of the Peyer's patch dome, giving the impression that physical trapping between the dome and adjacent villi during peristalsis had aided in their uptake. Although some particles up to 10 µm in diameter were observed within the Peyer's patch, microcapsules of less than 5 µm in diameter were taken up in greater numbers and were observed to progress deeper into the Peyer's patch over the time period examined. These results demonstrated that microcapsules of 1 to 5 µm in diameter are rapidly and selectively taken up from the gut lumen into the Peyer's patch. This suggested that microcapsules composed of biodegradable wall materials would serve as an effective means for the targeted delivery of antigens to the gut-associated lymphoreticular tissues for the induction of immunity of mucosal surfaces.

### 2. Oral immunization with antigen-loaded biodegradable microcapsules.

Microcapsules containing the haptenated protein antigen trinitrophenyl-keyhole limpet hemocyanin (TNP-KLH) were prepared using poly(DL-lactide-co-glycolide) copolymers as wall materials. These microcapsules were separated according to size and those in the range of 1 to 5 µm in diameter were selected for evaluation. This microcapsule contained 0.2% antigen by weight. Their ability to serve as an effective antigen delivery system when ingested was tested by administering 0.5 mL of a 10 mg/mL suspension (10 µg antigen) in bicarbonate-buffered sterile tap water via gastric incubation on 4 consecutive days. For comparative purposes an additional group of mice was orally Immunized in parallel with 0.5 mL of 20-µg/mL solution of unencapsulted TNP-KLH. Control mice were orally administered diluent only.

On days 14 and 28 following the final immunization, serum, saliva and gut secretions were obtained from 5 fasted mice in each group. These samples were tested in isotype-specific radioimmunoassays to determine the levels of TNP-specific and total antibodies of the IgM, IgG and IgA isotypes (Table 1). The samples of saliva and gut secretions contained antibodies which were almost exclusively of the IgA class. These results are consistent with previous studies and provide evidence that the procedures employed to collect these secretions do not result in contamination with serum. None of the immunization protocols resulted in significant changes in the total levels of immunoglobulins present in any of the fluids tested. Low but detectable levels of naturally-occurring anti-TNP antibodies of the IgM and IgG isotypes were detected in the serum and of the IgA isotype in the serum and gut secretions of sham immunized control mice. However, the administration of 30 µg of microencapsulated TNP-KLH in equal doses over 3 consecutive days resulted in the appearance of significant antigen-specific IgA antibodies over controls in the secretions. and of all isotypes in the serum by day 14 after immunication (see last column of Table 1). These antibody levels were increased further on day 28. In contrast, the oral administration of the same amount of unencapsulted antigen was ineffective at inducing specific antibodies of any isotype in any of the fluids tested.

### H. SIGNIFICANCE

These results are noteworthy in several respects. First, significant antigen-specific IgA antibodies are induced in the serum and mucosal secretions, a response which is poor or absent following the commonly used systemic immunization methods. Therefore, this immunization method would be expected to result in significantly enhanced immunity at the mucosa; the portal of entry or site of pathology for a number of bacterial and viral pathogens. Secondly, the microencapsulated antigen preparation was an effective immunogen when orally administered, while the same amount of unencapsulated antigen was not. Thus, the microencapsulation resulted in a dramatic increase in efficacy, presumably due to targeting of and increased uptake by the Peyer's patch. Thirdly, the inductive phase of the immune response appears to be of long duration. While systemic immunization with protein antigens in the absence of adjuvants is characterised by a peak in antibody levels in 7 to 14 days, the orally administered antigen-containing microcapsules induced responses which were higher at day 28 than day 14. This indicates that bioerosion of the wall materials and release of the antigen is taking place over an extended period of time, and thus inducing a response of greater duration.

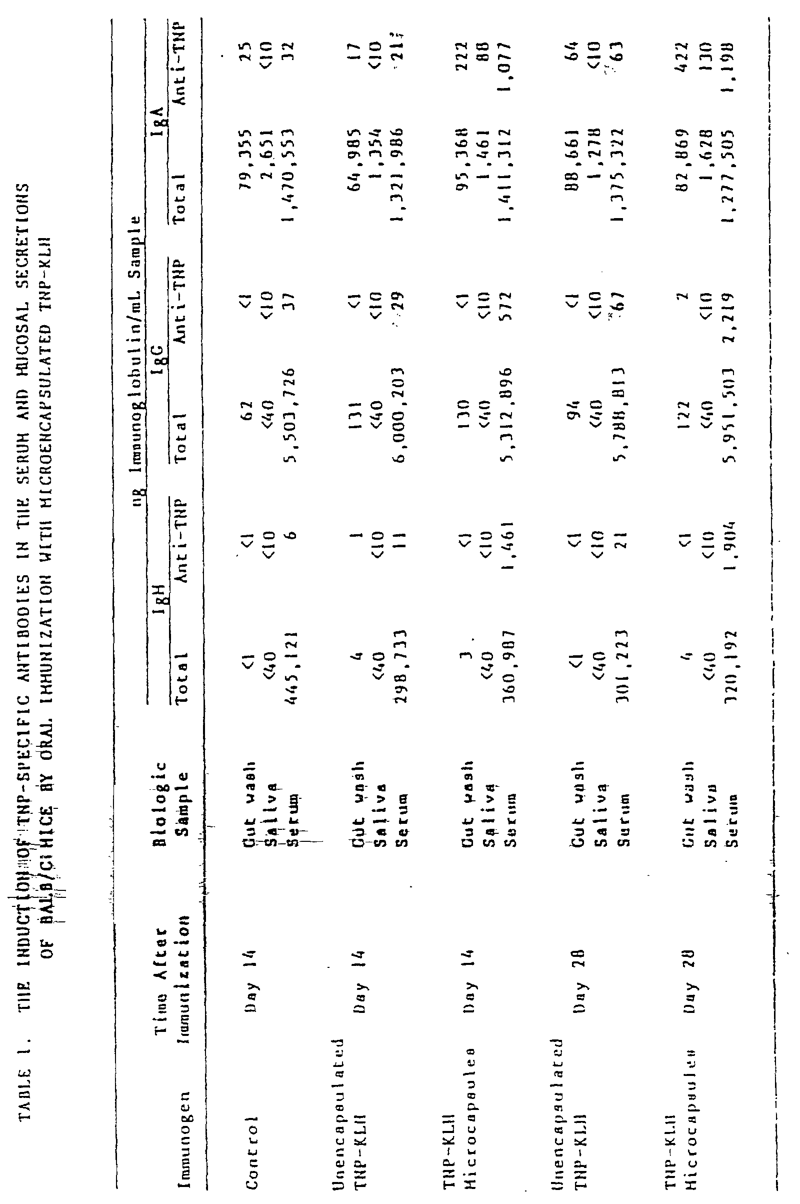

## Claims (Claims for the following Contracting State(s): DE, GB, FR, IT, NL, SE, LI, CH, BE, AT, LU)

1. An oral composition for immunisation to be administered to animals including humans, and capable of delivering an antigen to the Peyer's patch of said animal, comprising an effective amount of the antigen encapsulated in a biodegradable, biocompatible excipient so as to form microcapsules having a size less than or equal to 10 µm, being capable of being taken up selectively by the Peyer's patch and capable of passing through the gastrointestinal tract without degradation.

2. The composition according to claim 1 in which the microcapsules have a size of 1 µm to 10 µm.

3. The composition as claimed in claim 1 or 2, wherein the biodegradable, biocompatible excipient comprises first and second copolymer excipients having different copolymer ratios.

4. The composition as claimed in claim 3, wherein said microcapsule is 5 µm or less in diameter.

5. The composition as claimed in claim 3, wherein said microcapsule is 3 µm or less in diameter.

6. The composition as claimed in claim 5, wherein said excipient is a polymer.

7. The composition as claimed in claim 6, wherein said excipient is a copolymer.

8. The composition as claimed in claim 7, wherein said excipient is selected from the group consisting of poly(glycolic acid), copolymers of mixed DL-lactide and glycolide, copolyoxalates, polycaprolactone, poly(lactide-co-caprolactone), polyesteramides, polyorthoesters and poly(β-hydroxybutyric acid).

9. The composition as claimed in claim 8, wherein said copolymer excipient is poly(DL-lactide-co-glycolide).

10. The composition as claimed in claim 9, wherein said copolymer excipient has mole ratios of lactide to glycolide of 50:50 to 90:10, respectively.

11. The composition as claimed in claim 1 or 2, wherein at least one said antigen is a protozoal, viral, fungal or bacterial antigen.

12. The composition as claimed in claim 11, wherein said antigen is trinitrophenyl keyhole limpet hemocyanin.

13. The composition as claimed in claim 12, wherein said microcapsules are of diameter from between 1 to 5 µm, said antigen is trinitrophenyl keyhole limpet hemocyanin, and said excipient is 50:50 poly (DL-lactide-co-glycolide).

14. The composition as claimed in claim 1 or 2, wherein said antigen is a toxoid.

15. The composition as claimed in claim 14, wherein said toxoid is a toxoid of staphylococcal enterotoxin.

16. An oral composition for immunization to be administered to an animal, especially a human, for providing systemic and mucosal immunity in said animal, comprising an effective amount of an antigen encapsulated in a biodegradable, biocompatible excipient so as to form microcapsules of less than or equal to 10 µm in size, being capable of being taken up selectively by the Peyer's patch and capable of passing through the gastrointestinal tract without degradation and capable of delivering said antigen to the Peyer's patch of said animal.

17. The composition according to claim 16 in which the microcapsules have a size of 1 to 10 µm.

## Claims (Claims for the following Contracting State(s): ES, GR)

1. A method for preparing an oral composition for immunisation to be administered to animals including humans, and capable of delivering an antigen to the Peyer's patch of said animal, comprising encapsulating an effective amount of the antigen in a biodegradable, biocompatible excipient so as to form microcapsules having a size less than or equal to 10 µm, being capable of being taken up selectively by the Peyer's patch and capable of passing through the gastrointestinal tract without degradation.

2. The method according to claim 1 in which the microcapsules have a size of 1 µm to 10 µm.

3. The method as claimed in claim 1 or 2, wherein the biodegradable, biocompatible excipient comprises first and second copolymer excipients having different copolymer ratios.

4. The method as claimed in claim 3, wherein said microcapsule is 5 µm or less in diameter.

5. The method as claimed in claim 3, wherein said microcapsule is 3 µm or less in diameter.

6. The method as claimed in claim 5, wherein said excipient is a polymer.

7. The method as claimed in claim 6, wherein said excipient is a copolymer.

8. The method as claimed in claim 7, wherein said excipient is selected from the group consisting of poly(glycolic acid), copolymers of mixed DL-lactide and glycolide, copolyoxalates, polycaprolactone, poly(lactide-co-caprolactone), polyesteramides, polyorthoesters and poly(β-hydroxybutyric acid).

9. The method as claimed in claim 8, wherein said copolymer excipient is poly(DL-lactide-co-glycolide).

10. The method as claimed in claim 9, wherein said copolymer excipient has mole ratios of lactide to glycolide of 50:50 to 90:10, respectively.

11. The method as claimed in claim 1 or 2, wherein at least one said antigen is a protozoal, viral, fungal or bacterial antigen.

12. The method as claimed in claim 11, wherein said antigen is trinitrophenyl keyhole limpet hemocyanin.

13. The method as claimed in claim 12, wherein said microcapsules are of diameter from between 1 to 5 µm, said antigen is trinitrophenyl keyhole limpet hemocyanin, and said excipient is 50:50 poly (DL-lactide-co-glycolide).

14. The method as claimed in claim 1 or 2, wherein said antigen is a toxoid.

15. The method as claimed in claim 14, wherein said toxoid is a toxoid of staphylococcal enterotoxin.

16. A method for preparing an oral composition for immunisation to be administered to an animal, especially a human, for providing systemic and mucosal immunity in said animal, comprising encapsulating an effective amount of an antigen in a biodegradable, biocompatible excipient so as to form microcapsules of less than or equal to 10 µm in size, being capable of being taken up selectively by the Peyer's patch and capable of passing through the gastrointestinal tract without degradation and capable of delivering said antigen to the Peyer's patch of said animal.

17. The method according to claim 16 in which the microcapsules have a size of 1 to 10 µm.

## Patentansprüche (Patentansprüche für folgende(n) Vertragsstaat(en): DE, GB, FR, IT, NL, SE , LI, CH, BE , AT, LU)

1. Orale zur Immunisierung Tieren einschließlich des Menschen zu verabreichende und zur Freisetzung eines Antigens in die Peyer-Plaques des Tieres befähigte Zusammensetzung, die eine wirksame Menge des Antigens aufweist, das in einen biologisch abbaubaren, biokompatiblen Arzneimittelträger so verkapselt ist, dass Mikrokapseln mit einer Größe kleiner oder gleich 10 µm gebildet werden, die von den Peyer-Plaques selektiv aufgenommen werden können und den Gastrointestinaltrakt ohne Abbau passieren können.

2. Zusammensetzung nach Anspruch 1, bei der die Mikrokapseln eine Größe von 1 bis 10 µm aufweisen.

3. Zusammensetzung nach Anspruch 1 oder 2, bei der der biologisch abbaubare und biologisch verträgliche Arzneimittelträger einen ersten und einen zweiten Copolymerträger mit unterschiedlichen Copolymerverhältnissen umfasst.

4. Zusammensetzung nach Anspruch 3, bei der die Mikrokapsel einen Durchmesser von 5 µm oder darunter aufweist.

5. Zusammensetzung nach Anspruch 3, bei der die Mikrokapsel einen Durchmesser von 3 µm oder darunter aufweist.

6. Zusammensetzung nach Anspruch 5, bei der der Arzneimittelträger ein Polymer ist.

7. Zusammensetzung nach Anspruch 5, bei der der Arzneimittelträger ein Copolymer ist.

8. Zusammensetzung nach Anspruch 7, bei der der Arzneimittelträger ausgewählt ist aus der Gruppe bestehend aus Polyglycolsäure, Copolymeren aus einem Gemisch von DL-Lactid und Glycolid, Copolyoxalaten, Polycaprolacton, Poly(lactid-co-caprolacton), Polyesteramiden, Polyorthoestern und Poly(β-hydroxybuttersäure).

9. Zusammensetzung nach Anspruch 8, bei der der Copolymerarzneimittelträger Poly(DL-lactid-co-glycolid) ist.

10. Zusammensetzung nach Anspruch 9, bei der Copolymerarzneimittelträger Molverhältnisse von Lactid zu Glycolid von 50:50 bis 90:10 aufweist.

11. Zusammensetzung nach Anspruch 1 oder 2, bei der wenigstens ein Antigen ein Protozoen-, Viren-, Pilz- oder Bakterienantigen ist.

12. Zusammensetzung nach Anspruch 11, bei der das Antigen Trinitrophenyl-Napfschnecken (KLH)-Hämocyanin ist.

13. Zusammensetzung nach Anspruch 12, bei der die Mikrokapseln einen Durchmesser von 1 bis 5 µm aufweisen, das Antigen Trinitrophenyl-Napfschnecken-(KLH)-Hämocyanin ist und der Arzneimittelträger Poly(DL-lactid-co-glycolid) 50:50 ist.

14. Zusammensetzung nach Anspruch 1 oder 2, bei der das Antigen ein Toxoid ist.

15. Zusammensetzung nach Anspruch 14, bei der das Toxoid ein Toxoid eines Staphylokokkenenterotoxins ist.

16. Orale Zusammensetzung zur Immunisierung, die einem Tier, insbesondere einem Menschen zu verabreichen ist, um systemische Immunität und Schleimhautimmunität bei diesem Tier zu schaffen, die eine wirksame Menge eines Antigens enthält, das in einen biologisch abbaubaren, biokompatiblen Arzneimittelträger so verkapselt ist, dass Mikrokapseln einer Größe kleiner oder gleich 10 µm entstehen, die selektiv von den Peyer-Plaques aufgenommen werden und den Gastrointestinaltrakt ohne Abbau passieren können und befähigt sind, das Antigen in den Peyer-Plaques des Tieres abzugeben.

17. Zusammensetzung nach Anspruch 16, bei der die Mikrokapseln eine Größe von 1 bis 10 µm haben.

## Patentansprüche (Patentansprüche für folgende(n) Vertragsstaat(en): ES, GR)

1. Verfahren zur Herstellung einer oralen, Tieren einschließlich Menschen zur Immunisierung zu verabreichenden und zur Freisetzung eines Antigens in die Peyer-Plaques des Tieres befähigten Zusammensetzung, das beinhaltet, dass eine wirksame Menge des Antigens in einen biologisch abbaubaren, biokompatiblen Arzneimittelträger so verkapselt wird, dass Mikrokapseln mit einer Größe kleiner oder gleich 10 µm gebildet werden, die selektiv in die Peyer-Plaques aufgenommen werden und den Gastrointestinaltrakt ohne Abbau passieren können.

2. Verfahren nach Anspruch 1, bei dem die Mikrokapseln eine Größe von 1 bis 10 µm aufweisen.

3. Verfahren nach Anspruch 1 oder 2, bei dem der biologisch abbaubare und biologisch verträgliche Arzneimittelträger einen ersten und einen zweiten Copoplymerträger mit unterschiedlichen Copolymerverhältnissen umfasst.

4. Verfahren nach Anspruch 3, bei dem die Mikrokapsel einen Durchmesser von 5 µm oder darunter aufweist.

5. Verfahren nach Anspruch 3, bei dem die Mikrokapsel einen Durchmesser von 3 µm oder darunter aufweist.

6. Verfahren nach Anspruch 5, bei dem der Arzneimittelträger ein Polymer ist.

7. Verfahren nach Anspruch 6, wobei der Arzneimittelträger ein Copolymer ist.

8. Verfahren nach Anspruch 7, bei dem der Arzneimittelträger ausgewählt ist aus der Gruppe bestehend aus Polyglycolsäure, Copolymeren aus einem Gemisch von DL-Lactid und Glycolid, Copolyoxalaten, Polycaprolacton, Poly(lactid-co-caprolacton), Polyesteramiden, Polyorthoestern und Poly(β-hydroxybuttersäure).

9. Verfahren nach Anspruch 8, bei dem der Copolymerarzneimittelträger Poly(DL-lactid-co-glycolid) ist.

10. Verfahren nach Anspruch 9, bei dem der Copolymerarzneimittelträger Molverhältnisse von Lactid zu Glycolid von 50:50 bis 90:10 aufweist.

11. Verfahren nach Anspruch 1 oder 2, bei dem wenigstens ein Antigen ein Protozoen-, Viren-, Pilz- oder Bakterienantigen ist.

12. Verfahren nach Anspruch 11, wobei das Antigen Trinitrophenyl-Napfschnecken (KLH)-Hämocyanin ist.

13. Verfahren nach Anspruch 12, wobei die Mikrokapseln einen Durchmesser von 1 bis 5 µm aufweisen, das Antigen Trinitrophenyl-Napfschnecken-(KLH)-Hämocyanin ist und der Arzneimittelträger Poly(DL-lactid-co-glycolid) 50:50 ist.

14. Verfahren nach Anspruch 1 oder 2, wobei das Antigen ein Toxoid ist.

15. Verfahren nach Anspruch 14, wobei das Toxoid ein Toxoid eines Staphylokokkenenterotoxins ist.

16. Verfahren zur Herstellung einer oralen, einem Tier und insbesondere einem Menschen zur Immunisierung zu verabreichende Zusammensetzung, um systemische Immunität und Schleimhautimmunität bei dem Tier zu schaffen, das beinhaltet, dass eine wirksame Menge eines Antigens in einen biologisch abbaubaren, biokompatiblen Arzneimittelträger so verkapselt wird, dass Mikrokapseln mit einer Größe kleiner oder gleich 10 µm entstehen, die selektiv von den Peyer-Plaques aufgenommen werden können und den Gastrointestinaltrakt ohne Abbau passieren und befähigt sind, das Antigen in den Peyer-Plaques des Tieres abzugeben.

17. Verfahren nach Anspruch 16, bei dem die Mikrokapseln eine Größe von 1 bis 10 µm haben.

## Revendications (Revendications pour l'(les) Etat(s) contractant(s) suivant(s): DE, GB, FR, IT, NL, SE, LI, CH, BE, AT, LU)

1. Composition orale pour immunisation à administrer aux animaux, y compris les humains, et capable de délivrer un antigène au tampon de Peyer dudit animal, comprenant une quantité efficace d'antigène encapsulé dans un excipient biodégradable biocompatible de manière à former des microcapsules ayant une dimension inférieure ou égale à 10 µm étant capable d'être absorbé sélectivement par le tampon de Peyer et capable de passer à travers l'appareil gastro-intestinal sans dégradation.

2. Composition selon la revendication 1, dans laquelle les microcapsules ont une dimension de 1 à 10 µm.

3. Composition selon l'une des revendications 1 ou 2, dans laquelle l'excipient biodégradable et biocompatible comprend un premier et second excipients copolymères ayant différents ratios de copolymère.

4. Composition selon la revendication 3, dans laquelle ladite microcapsule a un diamètre de 5 µm ou moins.

5. Composition selon la revendication 3, dans laquelle ladite microcapsule a un diamètre de 3 i£m ou moins.

6. Composition selon la revendication 5, dans laquelle ledit excipient est un polymère.

7. Composition selon la revendication 5, dans laquelle l'excipient est un copolymère.

8. Composition selon la revendication 7, dans laquelle ledit excipient est choisi dans le groupe consistant en acides polyglycoliques, les copolymères de mélanges DL-lactide et glycolide, les copolyoxalates, le polycaprolactone, le poly(lactide-cocaprolactone), les polyesteraramides, les polyorthoesters et l'acide poly(β-hydroxybutyrique).

9. Composition selon la revendication 8, dans laquelle ledit excipient copolymère est le poly(DL-lactide-coglycolide).

10. Composition selon la revendication 9, dans laquelle ledit copolymère a un ratio molaire lactide/glycolide de 50/50 à 90/10 respectivement.

11. Composition selon l'une des revendications 1 et 2, dans laquelle au moins l'un desdits antigènes est un antigène protozoaire, viral, fongique ou bactérien.

12. Composition selon la revendication 11, dans laquelle l'antigène est la trinitrophényl hémocyanine à entrée molle.

13. Composition selon la revendication 12 dans laquelle lesdites microcapsules ont un diamètre compris entre de 1 et 5 µm ledit antigène étant la trinitrophényl hémocyanine molle et ledit excipient 50/50 poly(DL-lactide-coglycolide).

14. Composition selon l'une des revendications 1 ou 2, dans laquelle ledit antigène est un toxoïde.

15. Composition selon la revendication 14, dans laquelle ledit toxoïde est un toxoïde de l'entérotoxine staphylococcique.

16. Composition orale pour immunisation à administrer à un animal, spécialement à un humain, pour produire une immunité systémique et muqueuse dudit animal, comprenant une quantité efficace d'antigène encapsulée dans un excipient biodégradable et biocompatible de manière à former des microcapsules de dimensions inférieures ou égales à 10 µm, capable d'être absorbés sélectivement par le tampon de Payer et capable de passer à travers l'appareil gastro-intestinal sans dégradation et capable de délivrer ledit antigène au tampon de Payer dudit animal.

17. Composition selon la revendication 16, dans laquelle les microcapsules ont une dimension de 1 à 10 µm.

## Revendications (Revendications pour l'(les) Etat(s) contractant(s) suivant(s): ES, GR)

1. Méthode de préparation d'une composition orale pour immunisation à administrer aux animaux, y compris les humains, et capable de délivrer un antigène au tampon de Peyer dudit animal, comprenant une quantité efficace d'antigène encapsulé dans un excipient biodégradable biocompatible de manière à former des microcapsules ayant une dimension inférieure ou égale à 10 µm étant capable d'être absorbé sélectivement par le tampon de Payer et capable de passer à travers l'appareil gastro-intestinal sans dégradation.

2. Méthode selon la revendication 1, dans laquelle les microcapsules ont une dimension de 1 à 10 µm.

3. Méthode selon l'une des revendications 1 ou 2, dans laquelle l'excipient biodégradable et biocompatible comprend un premier et second excipients copolymères ayant différents ratios de copolymère.

4. Méthode selon la revendication 3, dans laquelle ladite microcapsule a un diamètre de 5 µm ou moins.

5. Méthode selon la revendication 3, dans laquelle ladite microcapsule a un diamètre de 3 µm ou moins.

6. Méthode selon la revendication 5, dans laquelle ledit excipient est un polymère.

7. Méthode selon la revendication 6, dans laquelle l'excipient est un copolymère.

8. Méthode selon la revendication 7, dans laquelle ledit excipient est choisi dans le groupe consistant en acides polyglycoliques, les copolymères de mélanges DL-lactide et glycolide, les copolyoxalates, le polycaprolactone, le poly(lactide-cocaprolactone), les polyesteraramides, les polyorthoesters et l'acide poly(β-hydroxybutyrique).

9. Méthode selon la revendication 8, dans laquelle ledit excipient copolymère est le poly(DL-lactide-coglycolide).

10. Méthode selon la revendication 9, dans laquelle ledit copolymère a un ratio molaire lactide/glycolide de 50/50 à 90/10 respectivement.

11. Méthode selon l'une des revendications 1 et 2, dans laquelle au moins l'un desdits antigène est un antigène protozoaire, viral, fongique ou bactérien.

12. Méthode selon la revendication 11 dans laquelle l'antigène est la trinitrophényl hémocyanine à entrée molle.

13. Méthode selon la revendication 12 dans laquelle lesdites microcapsules ont un diamètre compris entre de 1 et 5 µm ledit antigène étant la trinitrophényl hémocyanine molle et ledit excipient 50/50 poly(DL-lactide-coglycolide).

14. Méthode selon l'une des revendications 1 ou 2 dans laquelle ledit antigène est un toxoïde.

15. Méthode selon la revendication 14 dans laquelle ledit toxoïde est un toxoïde de l'entérotoxine staphylolococcique.

16. Composition orale pour immunisation à administrer à un animal, spécialement à un humain, pour produire une immunité systémique et muqueuse dudit animal, comprenant une quantité efficace d'antigène encapsulée dans un excipient biodégradable et biocompatible de manière à former des microcapsules de dimensions inférieures ou égales à 10 µm, capable d'être absorbés sélectivement par le tampon de Payer et capable de passer à travers l'appareil gastro-intestinal sans dégradation et capable de délivrer ledit antigène au tampon de Payer dudit animal.

17. Méthode selon la revendication 16, dans laquelle les microcapsules ont une dimension de 1 à 10 µm.
